# EUROPEAN PATENT APPLICATION

(11) **EP 4 438 616 A1**
(43) Date of publication of application: **02.10.2024**
(21) Application number: 22897027.3
(22) Date of filing: 08.04.2022
(51) Int. Cl.: C07K 7/06, C07K 7/08, C07K 14/00, A61K 38/08, A61K 38/10, A61K 38/16, A61K 8/64, A61P 1/02, A61P 1/04, A61P 17/00, A61P 17/02, A61P 17/18, A61Q 19/00, A61Q 19/02, A61Q 19/08

(54) **NEW POLYPEPTIDE FOR PROMOTING TISSUE REPAIR, AND USE THEREOF**

(30) Priority: 25.11.2021 CN 202111418086
(71) Applicant: Jinan University, Guangzhou, Guangdong 510000 (CN)
(72) Inventor: YE, Wencai, Guangzhou, Guangdong 510000 (CN); WANG, Lei, Guangzhou, Guangdong 510000 (CN); ZHANG, Dongmei, Guangzhou, Guangdong 510000 (CN); LIU, Junshan, Guangzhou, Guangdong 510000 (CN); FAN, Chunlin, Guangzhou, Guangdong 510000 (CN); CAO, Jiaqing, Guangzhou, Guangdong 510000 (CN)
(74) Representative: Schulz Junghans Patentanwälte PartGmbB
(86) International application number: PCT/CN2022/085715
(87) International publication number: WO 2023/092926

(57) **Abstract**

Disclosed in the present invention are a new polypeptide for promoting tissue repair, and the use thereof. The new polypeptide can promote the proliferation of human immortalized epidermal cells and the migration of human epidermal fibroblasts at a relatively low concentration, can promote the repair of wounds and ulcers, improves the aesthetic feeling of the skin, has low toxic side effects, has good application prospects, and can be used for preparing a drug and a medical instrument for treating wound surfaces, scalds and ulcers or for preparing an everyday chemical for improving the aesthetic feeling of the skin.

## Description

### Field of the Invention

The present disclosure relates to the field of drugs, medical instruments and everyday chemicals, in particular to a new polypeptide for promoting tissue repair and use thereof.

### Background of the Invention

With the development of society and the acceleration of the aging process, tissue traumas not only include bums, wounds and other traumas, but also include chronic wounds and ulcers such as gastric ulcers, oral ulcers, diabetic ulcers, autoimmune skin ulcers, venous stasis ulcers and pressure sores caused by prolonged bed rest. The chronic wounds and ulcers have complex pathogenesis, with prolonged course of disease, difficulty in treatment, and high treatment costs, imposing heavy physical, mental, and economic burdens on patients. At present, growth factor drugs are widely used in the treatment of the chronic wounds and ulcers, proving promising therapeutic effects. Specifically, epidermal growth factor, basic fibroblast growth factor, vascular endothelial growth factor, and the like, play significant roles in wounds and ulcers healing. However, the high preparation costs and poor stability of these endogenous factors limit their clinical applications. Therefore, it is imperative to seek and develop active substances with significant therapeutic activity, low production costs and good stability for the treatment of tissue traumas, chronic wounds and ulcers.

### Summary of the Invention

The present disclosure aims to overcome defects in the prior art and provide a new polypeptide capable of promoting tissue repair and use thereof.

To achieve the above purpose, the present disclosure adopts the following technical solution.

New polypeptides for promoting tissue repair, named **TRF1-TRF12**, and structures thereof are shown in formula I: 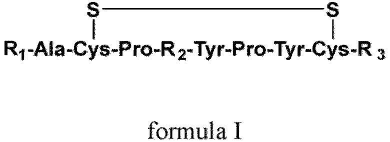 and in the formula I, R₁ = H or Ala or Gly or Ala-Ala- or Val-Ala-;
R₂ =Asn or Asp; and
R₃ = Ser or Tyr or -Gly-Ser-Tyr or -Gly-Ser-Tyr-Ala-Pro-Leu-Gly-Tyr or -Gly-Ser- Tyr-Ala-Pro-Leu-Gly- Tyr-His- V al-Arg or -Gly-Ser-Tyr-Ala-Pro-Leu-Gly-Tyr-His-Val-Arg-Glu-Tyr-Pro-Ala-Gly-Val-Ser-Ala-Ala.

Further, in a preferred embodiment of the present disclosure, new polypeptide compounds include but not limited to **TRF1-TRF12** as shown below:

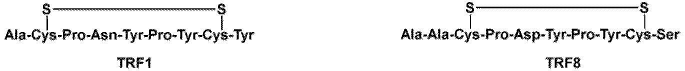

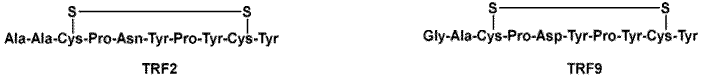

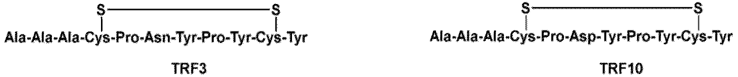

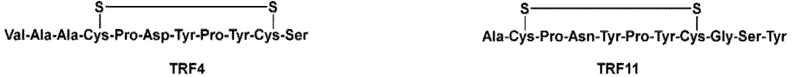

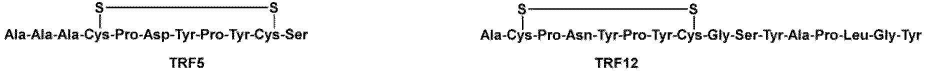

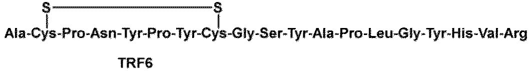

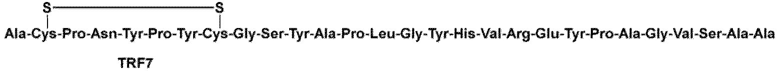

The new polypeptides **(TRF1-TRF12**) can be prepared by using methods known in the prior art, or can be chemically synthesized by using a polypeptide synthesizer, or can be biosynthesized by deducing nucleotide sequences from polypeptide sequences and then cloning them into an expression vector.

Another objective of the present disclosure is to disclose the use of the new polypeptides (**TRF1-TRF12**) in preparation of products for promoting tissue repair or improving aesthetic feeling of skin.

Preferably, the use of the new polypeptides (**TRF1-TRF12**) in preparation of products for treating wounds, scalds and ulcers or improving aesthetic feeling of skin is provided.

Preferably, the products preferably are drugs, medical instruments or everyday chemicals.

Preferably, the drugs, the medical instruments or the everyday chemicals contain an effective dose of one or more new polypeptides (**TRF1-TRF12**), or pharmaceutically acceptable salt thereof or a stereoisomer thereof, with balance of auxiliary materials or other compatible drugs.

The auxiliary materials refer to conventional excipients, such as solvents, disintegrants, flavoring agents, preservatives, colorants, binders, and the like.

The other compatible drugs refer to other natural drugs, chemical or biological medicines.

The drugs, the medical instruments or the everyday chemicals can be formulated as tablets, capsules, injections, liposome nanoparticles, controlled-release agents, gel medicinal extracts, ointments, liniments for external use, patches, creams, detergents, lotion, gels, toners, and the like.

The new polypeptide provided by the present disclosure has the following advantages and effects compared with the prior art:
(1) The new polypeptides (**TRF1-TRF12**) provided by the present disclosure have novel structures, with each polypeptide containing disulfide bond, making them structurally stable and resistant to degradation.
(2) The new polypeptides (**TRF1-TRF12)** provided by the present disclosure can significantly promote the proliferation of human immortalized epidermal cells (HaCAT) and the migration of human skin fibroblasts (HSF), exhibiting the effects of promoting tissue repair.
(3) The new polypeptides (**TRF1-TRF12)** provided by the present disclosure can be used for promoting tissue repair, and treating gastrointestinal ulcers and oral ulcers, and have effects of removing wrinkles, scars and pigmentations.
(4) The new polypeptides (**TRF1-TRF12)** provided by the present disclosure have low toxicity, with no obvious toxic side effects at an oral dose of 500 mg/kg.
(5) The new polypeptides (**TRF1-TRF12)** provided by the present disclosure can be chemically synthesized or biosynthesized, and are easy to prepare in large quantities.

### Brief Description of the Drawings

FIG. 1 shows a mass spectrum of a new polypeptide **TRF1**, where A is an ESI-MS spectrum thereof; B is an MS² spectrum thereof (*m*/*z* 50-580); and C is an MS² spectrum (*m*/*z* 580-1110).
FIG. 2 shows a mass spectrum of a new polypeptide **TRF2**, where A is an ESI-MS spectrum thereof; B is an MS² spectrum thereof *(m*/*z* 50-420); C is an MS² spectrum (*m*/*z* 390-810); and D is an MS² spectrum (*m*/*z* 810-1170).
FIG. 3 shows a mass spectrum of a new polypeptide **TRF3**, where A is an ESI-MS spectrum thereof; B is an MS² spectrum thereof (*m*/*z* 50-600); and C is an MS² spectrum (*m*/*z* 600-1300).
FIG. 4 shows a mass spectrum of a new polypeptide **TRF4**, where A is an ESI-MS spectrum thereof; B is an MS² spectrum thereof (*m*/*z* 50-510); and C is an MS² spectrum (*m*/*z* 510-1250).
FIG. 5 shows a mass spectrum of a new polypeptide **TRF5**, where A is an ESI-MS spectrum thereof; B is an MS² spectrum thereof (*m*/*z* 50-650); and C is an MS² spectrum (*m*/*z* 620-1200).
FIG. 6 shows a mass spectrum of a new polypeptide **TRF6**, where A is an ESI-MS spectrum thereof; B is an MS² spectrum thereof (*m*/*z* 50-400); C is an MS² spectrum (*m*/*z* 400-900); and D is an MS² spectrum (*m*/*z* 850-1800).
FIG. 7 shows a mass spectrum of a new polypeptide **TRF7**, where A is an ESI-MS spectrum thereof; B is an MS² spectrum thereof *(m*/*z* 50-400); C is an MS² spectrum (*m*/*z* 400-970); and D is an MS² spectrum (*m*/*z* 970-1700).
FIG. 8 shows a mass spectrum of a new polypeptide **TRF8**, where A is an ESI-MS spectrum thereof; B is an MS² spectrum thereof (*m*/*z* 50-620); and C is an MS² spectrum (*m*/*z* 620-1150).
FIG. 9 shows a mass spectrum of a new polypeptide **TRF9**, where A is an ESI-MS spectrum thereof; B is an MS² spectrum thereof (*m*/*z* 50-420); C is an MS² spectrum (*m*/*z* 390-810); and D is an MS² spectrum (*m*/*z* 800-1200).
FIG. 10 shows a mass spectrum of a new polypeptide **TRF10**, where A is an ESI-MS spectrum thereof; B is an MS² spectrum thereof (*m*/*z* 50-500); and C is an MS² spectrum (*m*/*z* 450-1250).
FIG. 11 shows a mass spectrum of a new polypeptide **TRF11**, where A is an ESI-MS spectrum thereof; B is an MS² spectrum thereof (*m*/*z* 50-420); C is an MS² spectrum (*m*/*z* 390-820); and D is an MS² spectrum (*m*/*z* 800-1270).
FIG. 12 shows a mass spectrum of a new polypeptide **TRF12**, where A is an ESI-MS spectrum thereof; B is an MS² spectrum thereof (*m*/*z* 50-380); C is an MS² spectrum (*m*/*z* 380-920); and D is an MS² spectrum (*m*/*z* 920-1560).
FIG. 13 shows an analysis of the effects of new polypeptides **TRF1-TRF12** on the proliferation of HaCAT cells.
FIG. 14 shows an analysis of the effects of new polypeptides **TRF1-TRF12** on the migration of HSF cells.

### Detailed Description of the Embodiments

The present disclosure will be further described in conjunction with examples and accompanying drawings, but the embodiments of the present disclosure are not limited thereto.

### Example 1 Synthesis of new polypeptides TRF1-TRF12

### (1) Solid-phase synthesis of the new polypeptide TRF1

1g of 0.3-0.5 mmol tyrosine (with Fmoc protection and side chain protection)-Wang resin was placed in a solid-phase synthesizer, and 20 mL of N,N-dimethylformamide was added to allow to completely swell the resin. The solution was then drained, and 20% piperidine (dissolved in the N,N-dimethylformamide) was added. The mixture was stirred for about 10 min and then drained. This process was repeated twice to ensure complete removal of the Fmoc group. The resin was washed with 20 mL of the N,N-dimethylformamide, the solvent was drained, and this washing step was repeated three times. Next, 0.5 mmol of cysteine with Fmoc protection and side chain protection was added, followed by an equal amount of HBTU/the N,N-dimethylformamide and N,N-diisopropylethylamine/the N,N-dimethylformamide solution. The reaction was carried out under nitrogen protection for 40-60 min; and unreacted drugs and reagents therein were eluted with the N,N-dimethylformamide, and then detected with ninhydrin. Subsequently, the same method was adopted to connect the remaining amino acids, and the polypeptide attached to the resin was obtained through deprotection, activation, condensation, washing and other steps. A cleavage buffer (trifluoroacetic acid/triisopropylsilane/pure water/1,2-ethanedithiol in a volume ratio of 94:2.5:2.5:1) was added to the resin-bound polypeptide for cutting for 2-3 h. Ice-cold diethyl ether was then added and centrifuged to obtain precipitates at a lower-layer. The precipitates were taken out, residual trifluoroacetic acid was removed. A certain quantity of iodine solution was added and stirred at room temperature for about 1 h to obtain a crude polypeptide.

The crude polypeptide was dissolved in an acetonitrile-water solution with a volume ratio of 20%, then filtrated and purified using a high performance liquid chromatography. Detection wavelengths were 214 nm, 254 nm and 280 nm, a mobile phase was acetonitrile-water (with a volume ratio of 10: 90-50: 50), and a flow rate was 1 mL/min. The target peak was collected, concentrated, and freeze-dried to obtain a pure polypeptide.

### (2) Solid-phase synthesis of the new polypeptides TRF2-TRF12

A synthesis process for the new polypeptides **TRF2-TRF12** is similar to that of **TRF1** described above. 1 g of 0.3-0.5 mmol amino acids (the first one at the carboxyl terminus) on Wang resin, with Fmoc protection and side chain protection, was placed in a solid-phase synthesizer. The amino acids in a sequence were sequentially obtained through deprotection, activation, condensation, washing and other steps. The polypeptide attached to the resin was then cleaved and purified to obtain a pure polypeptide.

### Example 2 Mass spectrometry identification of the new polypeptides TRF1-TRF12

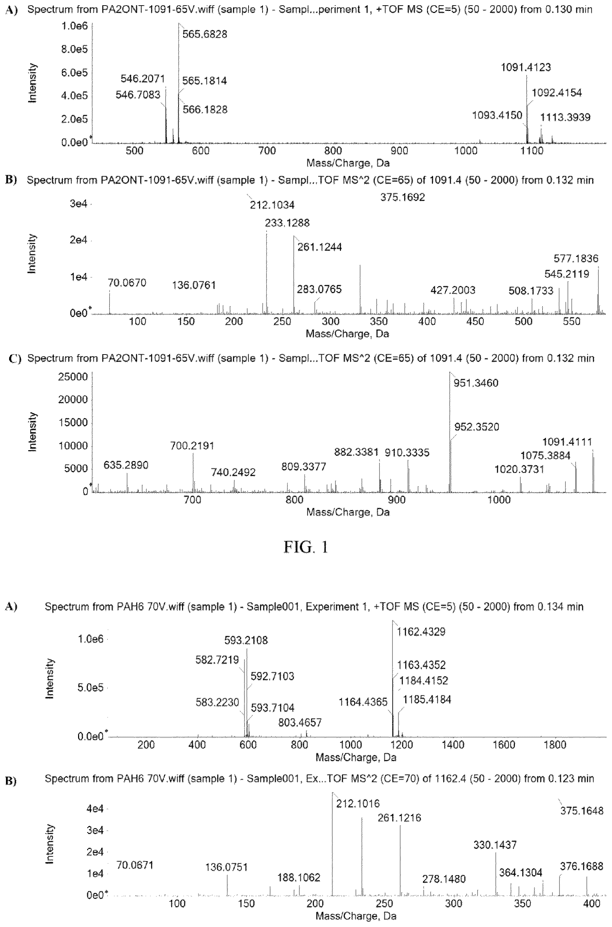

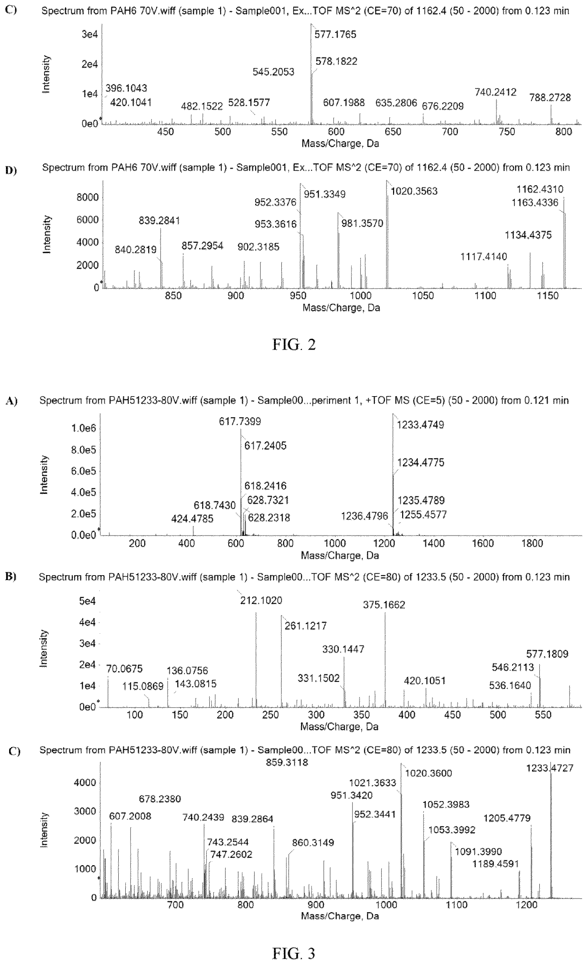

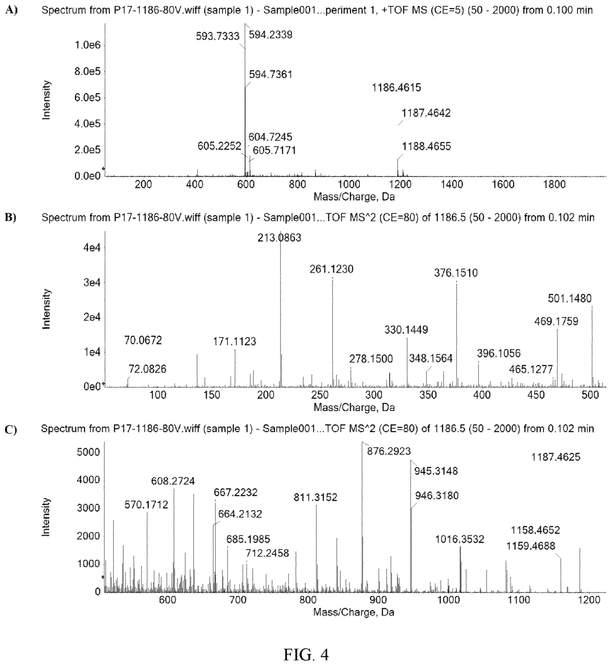

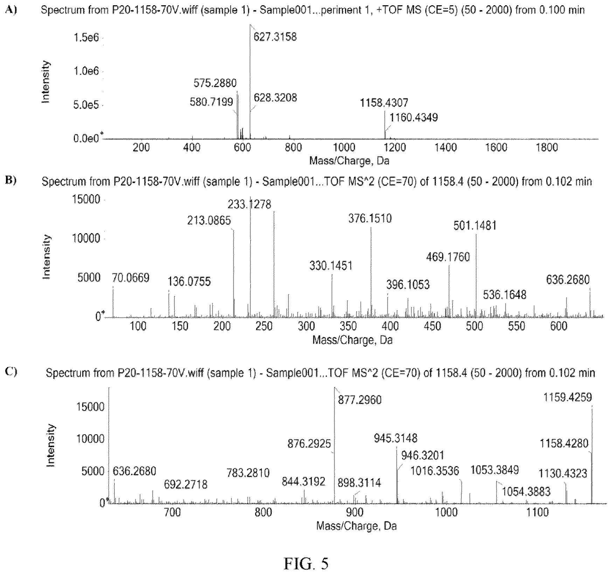

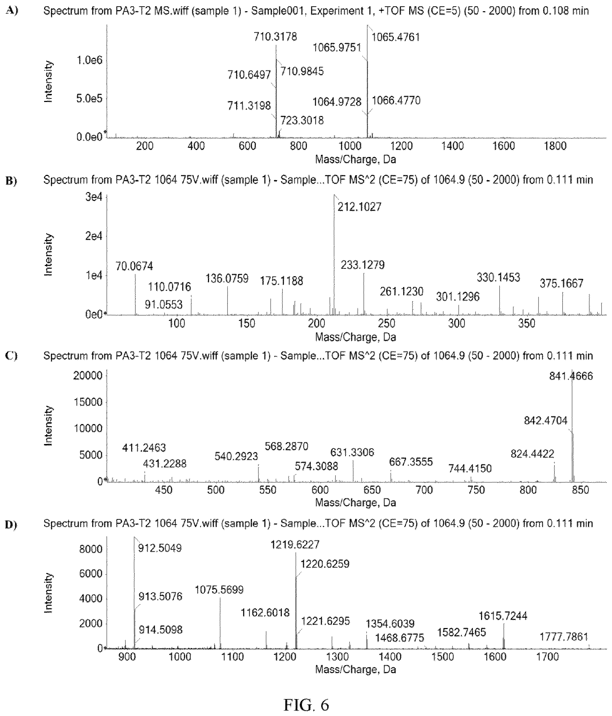

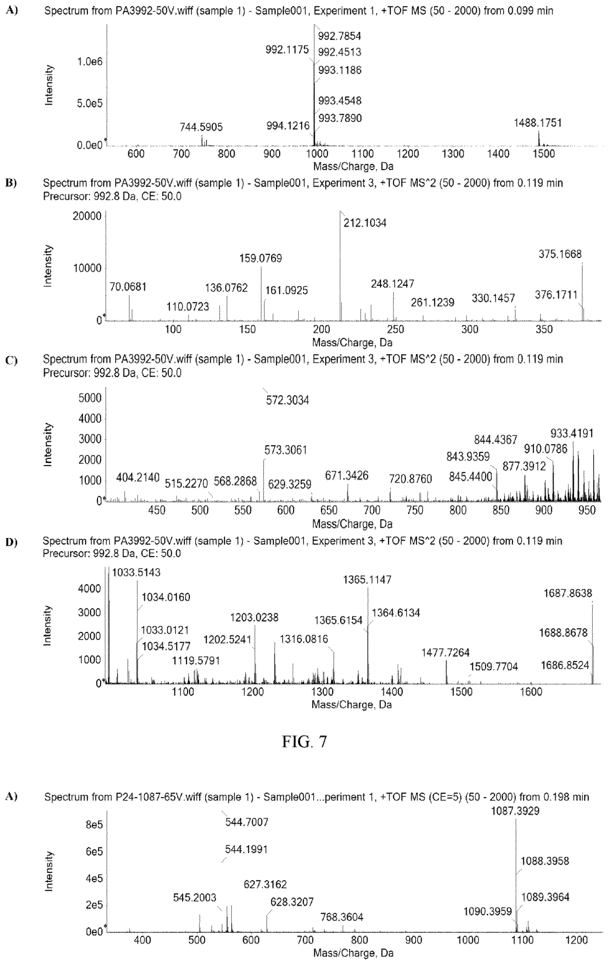

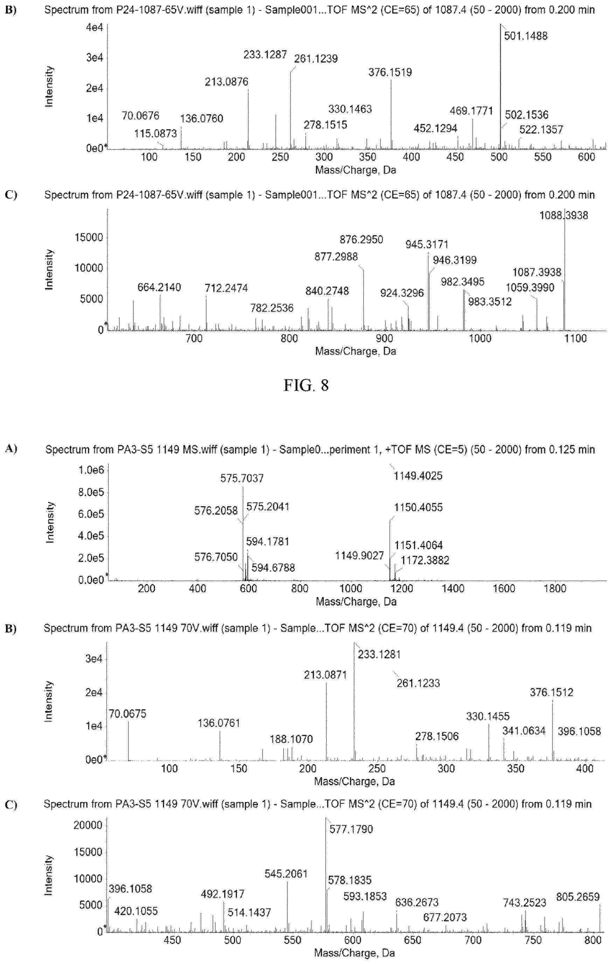

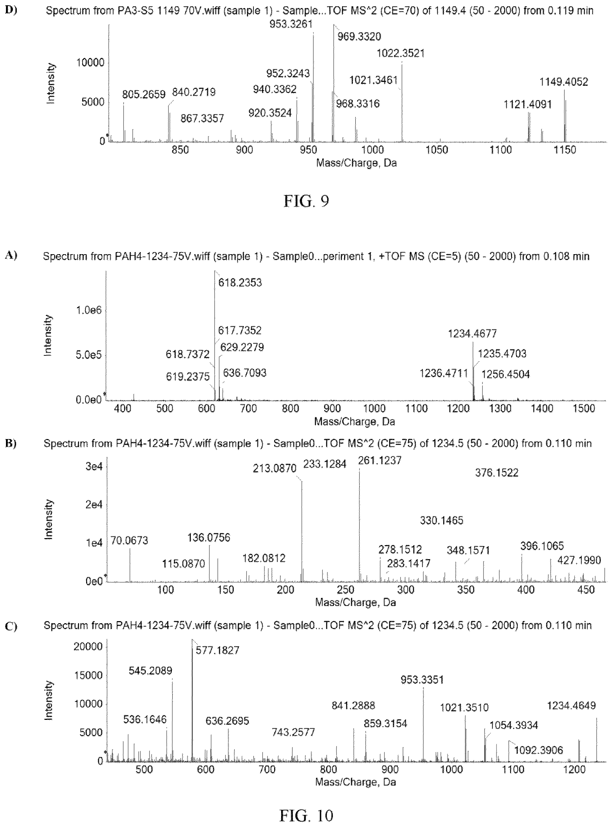

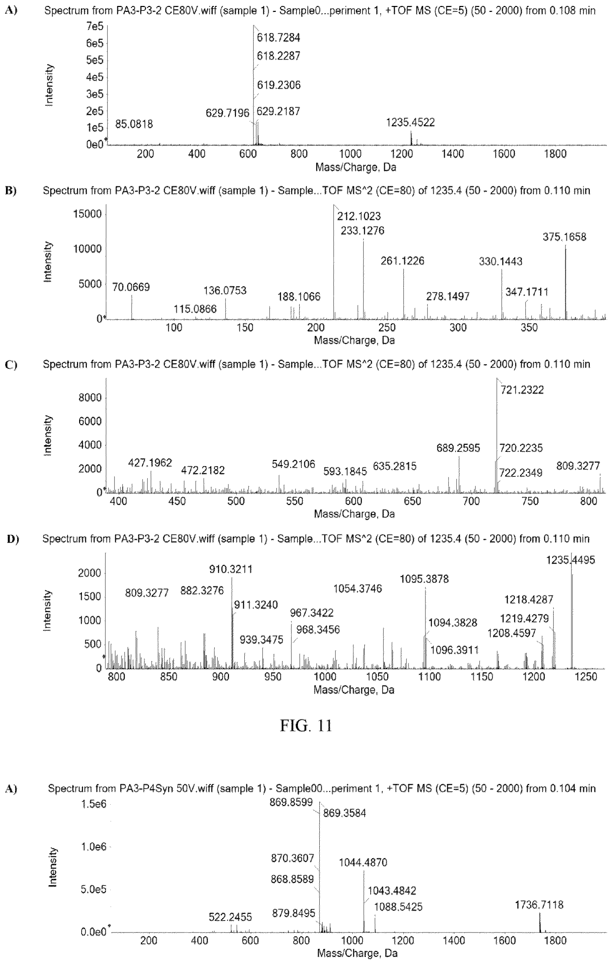

The polypeptides **TRF1-TRF12** were weighed and each dissolved in deionized water to prepare sample solutions with a concentration of 0.1 mg/mL. These sample solutions were then respectively injected into an AB Sciex TripleTOF^{®} 5600 + mass spectrometer for analysis, with a mass range (*m*/*z*) of 50-2000 Da, an acquisition mode of positive ion mode, a scanning mode of ion scanning selected, a capillary voltage of 3.0 kV, an ion source temperature of 110 °C, and a collision energy range of 50-80 V. Precursor ions were subjected to secondary scanning, and b and y ions thereof were analyzed based on secondary mass spectrum fragments to verify amino acid residue sequences of the new polypeptides, as shown in FIGs. 1-12.

### Example 3 Promoting effects of the new polypeptides TRF1-TRF12 on the proliferation of HaCAT cells

(1) Cell seeding: cells at a logarithmic growth phase were selected and counted after digestion. A desired number of cells were taken, and an appropriate amount of culture medium was added to the cells and diluted to 1 × 10⁴ cells/mL. The cells were then pipetted evenly using a multi-channel pipette. The cell solution was added to a 96-well plate (100 µL per well), and placed in a CO₂ incubator for incubation.
(2) Drug addition: the old culture mediums were removed after cell adhesion. A blank culture medium was added to a control group, EGF was added to a positive control group, and **TRF1-TRF12** with different concentrations were added to drug groups, respectively. PBS was added to the periphery of the well plate. Each group was tested in six replicate wells and incubated in the incubator for 48 h.
(3) MTT addition: the old culture mediums were resorbed, and a prepared MTT (protected from light) was added to each well (30 µL per well). The plates were then placed in the incubator and incubated for an additional 4 h to form formazan crystals.
(4) OD measurement: the MTT solution was removed, and 100 µL of DMSO was added to each well to completely dissolve the formazan crystals. An absorbance was measured at 595 nm using a multifunctional microplate reader. Cell viability was calculated, and each experiment was repeated at least 3 times.

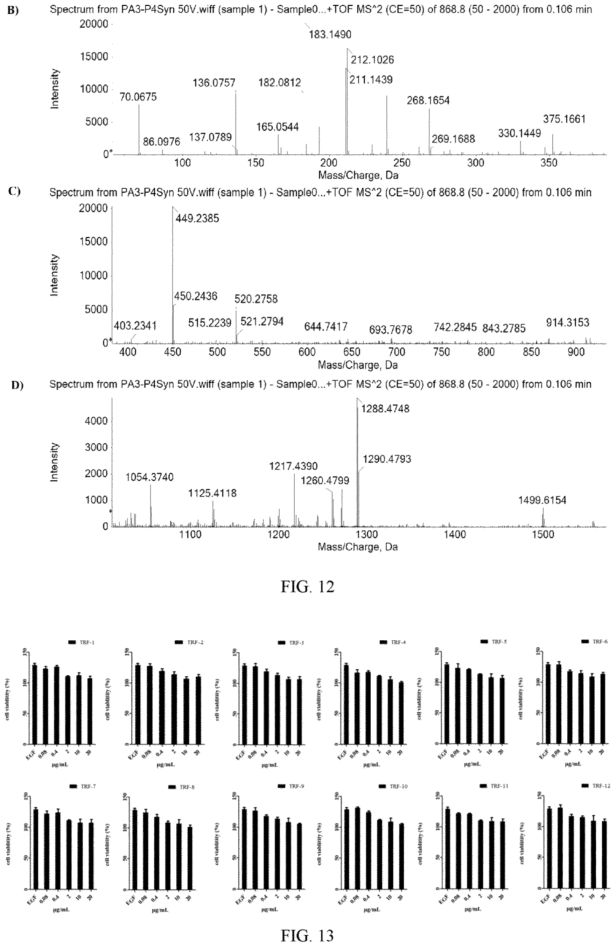

The results demonstrated that the new polypeptides **TRF1-TRF12** could significantly promote proliferation of HaCAT cells at low concentrations (0.08-2.00 µg/mL), with proliferation rates ranging from 114 ± 5% to 135 ± 3% (FIG. 13).

### Example 4 Promoting effects of the new polypeptides TRF1-TRF12 on the migration of HSF cells

(1) Cell seeding and drug addition: Transwell chambers were placed on a super clean bench for ultraviolet sterilization for 30 min, and then placed in a 24-well plate. After being counted after digestion, cells were diluted with culture mediums to a density of 2×10⁵ cells/mL. The test compounds **TRF1-TRF12** and positive control were added to the cell suspension to create drug-containing cell solutions at various concentrations. A volume of 100 µL of each drug-containing cell solution was added to the upper chamber of the treatment groups, while 100 µL of cell suspension was added to the blank control groups. Subsequently, 600 µL of blank culture medium was added to each lower chamber, and then placed in an incubator for incubation.
(2) Fixation, staining, and photography: Following the incubation period, the culture medium was discarded, and cells were washed with PBS. The cells in the lower chambers were fixed with 4% paraformaldehyde for approximately 30 min. After fixation, the cells were washed twice with the PBS, and 600 µL of staining solution was added to each lower chamber for 20 min. Crystal violets were then removed, and the cells were washed with the PBS. A cotton swab was used to gently remove non-migrated cells from an upper chamber membrane. After air drying, images were captured using a fluorescence inverted microscope. Five random areas from each chamber were photographed, and the average number of migrated cells was statistically analyzed using GraphPad Prism 6.0.

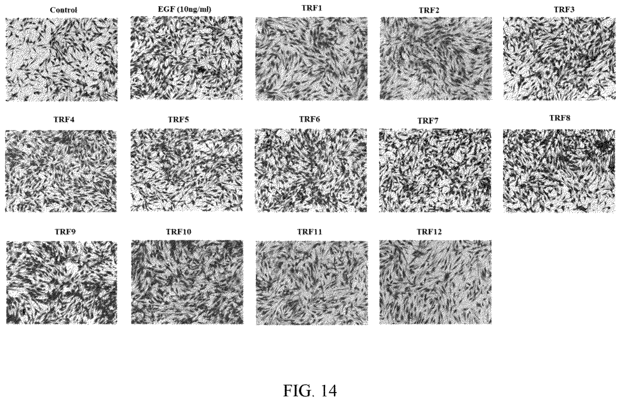

The results indicated that the new polypeptides **TRF1-TRF12** (at a concentration of 2 µg/mL) could significantly enhance the migration of HSF cells. After 48 h, the average number of migrated cells in the treatment groups ranged from 578 ± 9 to 396 ± 14, significantly higher than the control group (132 ± 10), with statistical significance (*P* < 0.05) (FIG. 14).

### Example 5 Repair effect of the new polypeptide TRF7 on skin wounds in mice

15 male Kunming mice aged 8-10 weeks (purchased from Guangdong Medical Experimental Animal Center) were selected and housed individually. The mice were randomly divided into four groups: a control group, a positive control group (Kangfuxin Liquid), a low-dose **TRF7** group (0.5 mg/mL), and a high-dose **TRF7** group (2.0 mg/mL). The mice were anesthetized, their backs were shaved and disinfected. The mice were subjected to anesthesia, after back shaving and disinfection. Circular wounds with a diameter of 6 mm were created on the exposed skin of the mice's backs using a punch biopsy tool, removing both the epidermis and dermis layers. Transparent oxygen-permeable dressings were applied to each wound to provide shielding and protection. The control group received normal saline. During each administration, the transparent oxygen-permeable dressings were gently lifted, and 0.1 mL of the respective drug solutions were applied to the wounds before new dressings were reapplied. This treatment was conducted for 10 days, with applications twice daily. The wound area was measured daily, and the mice were euthanized after 10 days of treatment. The wound healing rate was calculated using the formula: wound healing rate (%) = (S₁-Sₙ)/S₁ (where S₁ is a wound area on the first day, and Sₙ is a wound area on the n^{th} day). The results were shown in Table 1.

**Table 1 Statistical results of wound healing rates in mice**

| Group | | Number of mice | Healing rate (%) | | |
|---|---|---|---|---|---|
| | | | Day 3 | Day 5 | Day 10 |
| **TRF7** | 0.5 mg/mL | 10 | 22.3 ± 2.8* | 46.8 ± 5.2* | 82.1 ± 6.3* |
| | 2.0 mg/mL | 10 | 25.2 ± 2.6* | 50.1 ± 5.9* | 86.0 ± 7.7* |
| Kangfuxin Liquid | | 10 | 15.2 ± 5.1* | 25.7 ± 3.4* | 66.9 ± 7.3* |
| Saline | | 10 | 10.0 ± 3.1 | 18.8 ± 3.7 | 50.2 ± 5.0 |

| | | | | | |
|---|---|---|---|---|---|
| Note: Compared with the control group, **P* < 0.05 indicates a significant difference. | | | | | |

The results demonstrated that the new polypeptide **TRF7** could significantly promote the healing of skin wounds in mice, with a statistically significant difference compared to the control group, and better than that of Kangfuxin Liquid.

### Example 6 Repair effect of the new polypeptide TRF7 on skin scalds in mice

15 male Kunming mice aged 8-10 weeks (purchased from Guangdong Medical Experimental Animal Center) were housed individually with free access to food. Food intake was stopped one day before the experiment. A 13% Na₂S solution was used for depilation. A 20 g weight was heated in a water bath until boiling for 10 min, then clamped with forceps and quickly placed onto the skin of the mice for 5 seconds with slight pressure, creating a superficial second-degree scald model with an area of 2 cm × 2 cm. The next day, the scalds were treated with 0.1 mL of either **TRF7** (0.5 mg/mL or 2.0 mg/mL), saline, or Kangfuxin Liquid. Dressings were replaced once daily. Scald healing was observed on days 3, 5, and 10. The scald healing rate was calculated according to the method described in Example 5. The results are shown in Table 2.

**Table 2 Statistical results of scald healing rate in mice**

| Group | | Number of mice | Healing rate (%) | | |
|---|---|---|---|---|---|
| | | | Day 3 | Day 5 | Day 10 |
| **TRF7** | 0.5 mg/mL | 10 | 20.5 ± 3.9* | 38.1 ± 4.9* | 76.0 ± 8.7* |
| | 2.0 mg/mL | 10 | 23.9 ± 4.1* | 43.4 ± 5.1* | 81.5 ± 8.9* |
| Kangfuxin Liquid | | 10 | 14.9 ± 3.7* | 26.1 ± 3.8* | 60.9 ± 8.8* |
| Saline | | 10 | 11.4 ± 2.7 | 18.5 ± 3.5 | 46.2 ± 6.6 |

| | | | | | |
|---|---|---|---|---|---|
| Note: Compared with the control group, **P* < 0.05 indicates a significant difference. | | | | | |

The results demonstrated that the new polypeptide **TRF7** could significantly promote the healing of skin scalds in mice, with a statistically significant difference compared to the control group. Additionally, **TRF7** was found to be more effective than Kangfuxin Liquid.

### Example 7 Protective effect of the new polypeptide TRF7 on stress-induced gastric ulcer in rats

Test Method: 20 male SD rats weighing 180-210 g (purchased from Guangdong Medical Experimental Animal Center) were randomly divided into five groups: a blank group, a model group, a sucralfate group (1 g/kg, a positive drug group), a low-dose **TRF7** group (12.5 mg/kg), and a high-dose **TRF7** group (50.0 mg/kg). The treatment groups were subjected to intragastric administration for 7 consecutive days. 24 h before modeling, the rats were subjected to abrosia but fed with water. Except for the control group, modeling was performed by using a restraint water-immersion method, the rats in each group were fixed on a rat board, the rats with heads up were vertically immersed in a thermostatic water tank of (20°C) for 8 h, and a water surface was flush with xiphoids of the rats. After stress modeling was finished, the rats were euthanized through cervical dislocation, and then laparotomized, and pylorus ligation was performed. 2 mL of a formaldehyde solution of 10% by volume fraction was injected into stomach, cardias were ligated, and gastric bodies were taken out to be fixed in the formaldehyde solution for 15 min. The rats were cut open along greater curvatures of the stomach, rinsed with the normal saline, and then unfolded, damage to gastric mucosa was observed, and a gastric ulcer index was calculated. Calculating the ulcer index (UI) according to a Guth standard: punctate hemorrhage was scored as 1 point, linear hemorrhage with a length of < 1 mm was scored as 2 points, linear hemorrhage with a length of 1-2 mm was scored as 3 points, linear hemorrhage with a length of 2-4 mm was scored as 4 points, linear hemorrhage with a length of > 4 mm was scored as 5 points, and linear hemorrhage with a width of > 1 mm was scored double points. The results were shown in Table 3.

**Table 3 Statistical results of ulcer index of rats**

| Group | | Number of rats | Ulcer index |
|---|---|---|---|
| **TRF7** | 12.5 mg/kg | 10 | 28.1 ± 6.9* |
| | 50.0 mg/kg | 10 | 22.2 ± 6.5* |
| Sucralfate group | | 10 | 35.5 ± 8.7* |
| Model group | | 10 | 75.1 ± 13.8 |
| Blank group | | 10 | 8.9 ± 2.5 |

| | | | |
|---|---|---|---|
| Note: Compared with the model group, *P < 0.01 indicates a significant difference. | | | |

The results showed that the new polypeptide **TRF7** exerted a significant protective effect on stress-induced gastric ulcers in rats, with a statistically significant difference compared to the model group. Additionally, the protective effect of **TRF7** was superior to that of the positive control sucralfate.

### Example 8 Protective effect of the new polypeptide TRF7 on oral ulcers in rats

Test method: 15 healthy SD rats weighing 180-210 g (purchased from Guangdong Medical Experimental Animal Center) were randomly divided into a model group, a positive drug group (Kangfuxin Liquid), a low-dose **TRF7** group (0.5 mg/mL), and a high-dose **TRF7** group (2.0 mg/mL), and modeling was performed by a chemical calcination method. A plastic tube with a diameter of 5 mm was taken, a small cotton ball soaked in a 95% phenol solution was placed at one end of the plastic tube, a cotton end was fixed on left buccal mucosa of the anesthetized rat, a fixing state was kept for 60 seconds, and calcinated surfaces were washed with the normal saline. After 24 h, if it was observed that a circular ulcer was formed in a left experiment area, and if the circular ulcer has a yellow white abscess on the surface, has a clear boundary with periphery, has red, swollen and congested surrounding mucosa, and has an ulcer surface with a diameter being > 3 mm, the modelling was successful. 0.1 mL of a new polypeptide solution, 0.1 mL of normal saline, and 0.1 mL of Kangfuxin Liquid were dropwise added to the ulcer surfaces of rats in the experiment group, the ulcer surfaces of rats in the model group, and the ulcer surfaces of rats in the positive drug group, respectively, twice a day. Ulcer surface healing situations were observed after 1 day, 3 days, and 5 days. The scoring criteria for ulcers were as follows: the ulcers without congestion or edema and having an ulcer surface diameter being < 1 mm were scored as 1 point; the ulcers with mild congestion and edema and having an ulcer surface diameter being 1-2 mm were scored as 2 points; the ulcers with moderate congestion and edema and having an ulcer surface diameter being 2-3 mm were scored as 3 points; and the ulcers with severe congestion and edema and having an ulcer surface diameter being > 3 mm was scored as 4 points. The results were shown in Table 4.

**Table 4 Oral ulcer healing integrals of rats**

| Group | | Oral ulcer healing integral value | | | |
|---|---|---|---|---|---|
| | | Before administration | Day 1 | Day 3 | Day 5 |
| **TRF7** | 0.5 mg/mL | 4.8 ± 0.4 | 3.2 ± 0.5* | 2.8 ± 0.5* | 1.6 ± 0.4* |
| | 2.0 mg/mL | 4.7 ± 0.3 | 3.3 ± 0.6* | 2.8 ± 0.5* | 1.3 ± 0.3* |
| Kangfuxin Liquid (positive drug group) | | 4.9 ± 0.3 | 3.5 ± 0.4* | 3.0 ± 0.4* | 2.8 ± 0.3* |
| Saline (Model group) | | 5.1 ± 0.4 | 4.6 ± 0.5 | 3.9 ± 0.5 | 3.4 ± 0.4 |

| | | | | | |
|---|---|---|---|---|---|
| Note: Compared with the model group, **P* < 0.05 indicates a significant difference. | | | | | |

The results indicated that the new polypeptide **TRF7** could significantly promote the healing of oral ulcers of in rats, with a significant difference compared with the model group, and was more effective than Kangfuxin Liquid.

### Example 9 Acute toxicity test of the new polypeptides TRF1-TRF12

Test method: Kunming mice weighing 18-22 g (purchased from Guangdong Medical Laboratory Animal Center) were randomly divided into groups of 10 mice each. The mice were administered different doses of **TRF1-TRF12** intragastrically. The median lethal dose (LD₅₀) was calculated based on the survival rate of the mice. The LD₅₀ was calculated using the following formula: Median lethal dose (mg/kg) = (Dose at which half of the mice died/weight of the corresponding mice).

Test results: No deaths or significant adverse reactions were observed in the mice administered with the new polypeptides **TRF1-TRF12** at a dose of 500 mg/kg, proving relatively low toxicity of the new polypeptides. The LD₅₀ was determined to be greater than 500 mg/kg (Table 5).

**Table 5 Acute toxic effects of TRF1-TRF12 on mice**

| Sample | Dose (mg/kg) | Number of mice | Number of surviving mice after administration | LD₅₀ (mg/kg) |
|---|---|---|---|---|
| **TRF1-TRF12** | 100 | 10 | 10 | > 500 |
| | 200 | 10 | 10 | |
| | 500 | 10 | 10 | |

### Example 10 Skin safety evaluation of the new polypeptide TRF7

Subject population: A total of 20 subjects, comprising 10 females and 10 males aged 18-55, were selected. All subjects had healthy skin without any history of skin diseases or allergies, meeting the eligibility criteria for participation.

Test method: Qualified patch testers were selected, and a closed patch test method was adopted. Approximately 0.020-0.025 mL of the polypeptide **TRF7** (2 mg/mL) was applied to the patch testers, which were then affixed to the subjects' backs using specialized adhesive tape. The test patches were removed after 24 h, and skin reactions were observed at 0.5, 6, 12, 24, and 48 h post-removal. The results of the skin reactions were recorded according to the grading standards stipulated in the *Hygienic Standard for Cosmetics.*

Test results: All 20 subjects passed the patch test, with no adverse skin reactions observed at 0.5, 6, 12, 24, and 48 h post-application, indicating that the new polypeptide **TRF7** in the present disclosure is safe for skin use.

### Example 11 Preparation of tablets

0.1 g of the polypeptide **TRF7**, 40 g of lactose, 60 g of starch slurry, and 0.2 g of magnesium stearate were mixed together, sieved, and dried, and then pressed into tablets. Each tablet contained 0.001 g of **TRF7**.

### Example 12 Preparation of injections

0.1 g of the polypeptide **TRF7** and 50 g of propylene glycol were ground together, and then diluted with 100 mL of injection-grade water and mixed thoroughly. Subsequently, 9 g of sodium chloride was added and dissolved. Injection-grade water was added to bring the total volume to 1000 mL. The pH value was adjusted to 5.5-6.5, followed by filtration, filling, and sterilization to obtain 1000 injections.

### Example 13 Preparation of solid lipid nanoparticles

0.1 g of the polypeptide **TRF7** was mixed with 500 mg of soybean lecithin and dissolved in 25 mL of ethanol. Separately, 200 mg of stearic acid and 500 mg of soybean lecithin were dissolved in 25 mL of cyclohexane. The two solutions were then mixed and stirred evenly. The mixture was placed in a thermostatic water bath at 37°C and subjected to rotary evaporation under reduced pressure to remove the organic solvents, forming a uniform lipid film on the flask wall. The film was left in a vacuum dryer overnight to remove any remaining organic solvents. Separately, 3750 mg of polyethylene glycol monostearate was dissolved in 175 mL of water by stirring, and then added to the above film and sonicated for 10 min. The solution was brought to a final volume of 250 mL, resulting in a light-yellow transparent solution. This solution was freeze-dried to obtain a powder, which was then ground in a ball mill for 24 h to achieve nanoparticles with uniform size. The nanoparticles were thoroughly mixed and packaged, with each package containing 0.001 g of **TRF7**.

### Example 14 Preparation of controlled-release capsules

0.1 g of the polypeptide **TRF7** was combined with 40 g of lactose and 10 g of starch slurry, then loaded into a rotary granulator/coater to prepare granules. A plasticized ethylcellulose coating agent suspension, diluted to contain 15% by mass fraction of solids, was sprayed onto the rotating bed of polypeptide granules. During spraying, the granules were coated with a dispersion carrier film made from Poloxamer 188, forming sustained-release granules with an average particle size of about 450 µm. The granules were then mixed well and encapsulated into capsules, with each capsule containing 0.001 g of **TRF7**.

### Example 15 Preparation of external gel medicinal extracts

2 g of glycerin was weighed, followed by the addition of 0.75 g of polyacrylic acid and 0.1 g of aluminum hydroxide, which were thoroughly mixed. Then, 0.2 g of the polypeptide **TRF7** was added, and mixed thoroughly under vacuum conditions to obtain a mixture ①. Separately, 5 g of purified water was weighed, 0.06 g of lactic acid and 0.1 g of sodium carboxymethyl cellulose were dissolved in the purified water to obtain mixture ②. The mixture ② was added to the mixture ① and stirred thoroughly under vacuum conditions to obtain a medicated gel after a cross-linking reaction. The medicated gel was then applied with a layer thickness of approximately 1.0 mm, and cut into standard plaster sizes, with each plaster containing 0.02 g of **TRF7**, then dried and packaged.

### Example 16 Preparation of ointment

40 g of stearyl alcohol and 45 g of white vaseline were melted in a water bath and heated to 75°C for subsequent use. Separately, 1.46 g of sodium dodecyl sulfate, 0.025 g of methylparaben, 0.015 g of propylparaben, 13 g of propylene glycol, and 0.2 g of the polypeptide **TRF7** were successively dissolved in water and heated to 75°C. The melted stearyl alcohol and white vaseline at 75°C were then added to the solution and stirred until cool to obtain an ointment containing 0.2% of **TRF7.**

### Example 17 Preparation of liniment for external use

0.1 g of the polypeptide **TRF7** was dissolved in 45 mL of distilled water, then filtered to obtain a filtrate. 5 mL of glycerin and 1 mL of azone were added to the filtrate, and distilled water was added to bring the total volume to 50 mL, resulting in a liniment for external use containing 0.2% of **TRF7.**

### Example 18 Preparation of adhesive bandages

2 g of polyoxyl-40-stearate, 2 g of polysorbate 80, 1 g of poloxamer 188, 0.3 g of sodium dihydrogen phosphate, 0.1 g of ethyl hydroxybenzoate, and 50 mL of purified water were weighed and sterilized at 130°C for 30-60 min, then cooled to 30-40°C and stirred evenly to obtain a mixture ① Separately, 10 g of light liquid paraffin, 0.5 g of cetyl alcohol, 0.5 g of stearyl alcohol, 3 g of white vaseline, and 1 g of glyceryl monostearate were weighed, sterilized, and kept warm at 30-40°C to obtain a mixture ②. 0.1 g of the polypeptide **TRF7** was dissolved in 50 mL of distilled water, filtered, and added to the mixture ①, then stirred to obtain a mixture (3). The mixture ② was slowly added to the mixture (3), followed by 3 g of 15% ethanol, then quickly stirred. The pH value was adjusted to 6.0, and the temperature was maintained at 45°C. The mixture was sheared at high speed, emulsified for 5 min, stirred, and cooled to 30-35°C to obtain an off-white homogeneous cream. The cream was poured onto a feeding plate, coated onto an inner pad with a coating machine, and dried at a low temperature (45°C). The dried cream was then cut into the desired sizes to obtain medicated inner pads, which were attached to the middle of a base fabric, and release paper was attached to the adhesive parts at both ends of the base fabric.

### Example 19 Preparation of face cream

0.1 g of the polypeptide **TRF7** was weighed and set aside. Separately, 1 g of No. 26 white oil, 1 g of stearyl alcohol, 1 g of stearic acid, 0.5 g of monostearin, 0.05 g of 350 silicone oil, 0.5 g of GTCC, and 0.03 g of methylparaben were mixed and heated to 90°C to form the first semi-finished product. 4 g of deionized water, 0.1 g of the polypeptide **TRF7**, 0.3 g of polyoxyethylene lauryl ether, and 0.5 g of glycerin were added to the first semi-finished product, and heated to 80°C to form the second semi-finished product. The second semi-finished product was homogenized twice at 80°C (at a speed of 3000 rpm for 10 min each time), stirred for 30 min, cooled to 45°C to form a cream, and 0.005 g of Casson was added and mixed evenly to prepare a facial cream containing 1% of **TRF7**.

### Example 20 Preparation of facial masks

0.1 g of the polypeptide **TRF7** was dissolved in 10 mL of deionized water and filtered to obtain a filtrate. The filtrate was mixed with 0.3 g of carbomer moistened with glycerin, triethanolamine was added, and the pH value thereof was adjusted to 6-7. The mixture was then uniformly applied onto face mask paper to prepare masks containing 0.01 g of **TRF7** per mask.

### Example 21 Preparation of toner

0.1 g of the polypeptide **TRF7** was weighed and prepared to obtain a 0.2 mg/mL aqueous solution. Separately, 5 g of glycerin was added to 1 g of hyaluronic acid, dispersed, and dissolved in water while stirring evenly to obtain a hyaluronic acid solution. Then, 1 g of trehalose and 1 g of allantoin were dissolved in water and mixed with the hyaluronic acid solution to form a mixed solution. The polypeptide solution, 1 g of D-panthenol, 10 g of oat β-glucan, 10 g of 1,2-pentanediol and 0.05 g of preservative were sequentially added to the mixed solution and stirred evenly with water to obtain the toner.

### Example 22 Preparation of lotion

Step (1): 0.1 g of the polypeptide **TRF7** was dissolved to prepare a 0.2 mg/mL aqueous solution.
Step (2): 0.05 g of sodium hyaluronate was dissolved in water, dispersed, and stirred evenly to obtain a hyaluronic acid solution.
Step (3): 5 g of glycerin, 3 g of butylene glycol, 2 g of trehalose, 0.2 g of allantoin, and 0.1 g of thickener were dissolved in water and heated to 75°C.
Step (4): 2 g of jojoba oil, 3 g of hydrogenated polydecene, 2 g of polydimethylsiloxane, 3 g of caprylic/capric glycerides and 3 g of emulsifier were mixed and heated to 75°C while stirred evenly.
Step (5): The product obtained in step (3) was quickly poured into the product obtained in the step (4), homogenized for 3-5 min at a constant temperature, and then cooled.
Step (6): cooled down to below 60°C, then the aqueous solution obtained in step (1) and the hyaluronic acid solution obtained in step (2) were added and homogenized. Once cooled to below 40°C, preservative and fragrance were added to obtain the lotion.

### Example 23 Preparation of cleansing gel

0.1 g of the polypeptide **TRF7** was dissolved to prepare a 0.2 mg/mL polypeptide solution. The polypeptide solution, 2.5 g of glycerin, 3 g of decyl glucoside, 3 g of sodium cocoyl apple amino acid, 1.5 g of sodium laureth sulfate, 0.1 g of solubilizer, and 0.05 g of fragrance were sequentially added to water and stirred. Finally, 0.5 g of thickener was added and stirred until completely swollen to obtain the cleansing gel.

### Example 24 Evaluation on cosmetic effect of new polypeptide lotion

1.1 Experiment substances: the new polypeptide lotion prepared in Example 22 according to the present disclosure.
1.2 Subject population: 30 subjects aged 18-55 in total, with 18 females and 12 males. Skins of the subjects had wrinkles, pigmentations, dull complexion, as well as unattractive factors on the skin such as minimal trauma and post-surgery scars.
1.3 Test method: after the skin of the subjects was cleaned, the lotion prepared in Example 22 was applied to the skin, and effects of use were observed and felt.
1.4. The test evaluation results are shown below:

**Table 6 Summary of feedback on the new polypeptide TRF7 lotion (8 Weeks)**

| Evaluation Criteria | Evaluation results | | | | | |
|---|---|---|---|---|---|---|
| | Very Good | Relatively good | General | Relatively bad | Very bad | Other |
| Wrinkles | 19 | 6 | 5 | 0 | 0 | 0 |
| Scars | 20 | 5 | 5 | 0 | 0 | 0 |
| Pigmentatio ns | 19 | 7 | 4 | 0 | 0 | 0 |
| Skin radiance | 22 | 5 | 3 | 0 | 0 | 0 |
| Skin elasticity | 23 | 5 | 2 | 0 | 0 | 0 |

Subjects reported that using the new polypeptide **TRF7** lotion improved skin hydration, enhanced skin moisture retention and elasticity, and made the skin appear more radiant and supple. The product demonstrated effects in whitening, reducing wrinkles, scars, and pigmentation. No allergic reactions were observed among the subj ects.

### Example 25 Evaluation on cosmetic effect of new polypeptide facial masks

1.1 Experiment substances: The polypeptide facial masks prepared in Example 20 according to the present disclosure.
1.2 Subject population: 30 subjects aged 18-58 in total, with 21 females and 9 males. Facial skins of the subjects had wrinkles, pigmentations, dull complexion, as well as unattractive factors on the skin such as minimal trauma and post-surgery scars.
1.3 Test method: after the faces of the subjects were cleaned, the new polypeptide facial masks prepared in Example 20 were applied to the faces, once a day, and effects of use were observed and felt.
1.4. The test evaluation results are shown below:

**Table 7 Summary of feedback on the new polypeptide TRF7 facial masks (8 Weeks)**

| Evaluation Criteria | Evaluation results | | | | | |
|---|---|---|---|---|---|---|
| | Very Good | Relatively good | General | Relatively bad | Very bad | Other |
| Wrinkles | 20 | 6 | 4 | 0 | 0 | 0 |
| Scar | 20 | 5 | 5 | 0 | 0 | 0 |
| Pigmentations | 21 | 5 | 4 | 0 | 0 | 0 |
| Skin radiance | 20 | 4 | 6 | 0 | 0 | 0 |
| Skin elasticity | 20 | 6 | 4 | 0 | 0 | 0 |

Subjects reported that using the new polypeptide **TRF7** facial masks improved skin hydration, enhanced skin moisture retention and elasticity, and made the skin appear more radiant and supple. The product demonstrated effects in whitening, reducing wrinkles, scars, and pigmentation. No allergic reactions were observed among the subjects.

The above examples represent preferred implementations of the present disclosure but do not limit its scope. Any other changes, modifications, substitutions, combinations, or simplifications that do not deviate from the spirit and principles of the present disclosure should be equivalent substitution manners and are included in the scope of protection of the present disclosure.

## Claims

1. A new polypeptide for promoting tissue repair, wherein a structure of the new polypeptide is shown as follows: 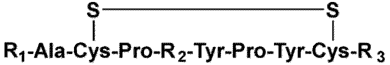 wherein: R₁ = H, Ala, Gly, Ala-Ala- or Val-Ala-;
R₂ =Asn or Asp;
R₃ = Ser, Tyr, -Gly-Ser-Tyr, -Gly-Ser-Tyr-Ala-Pro-Leu-Gly-Tyr, -Gly-Ser-Tyr-Ala-Pro-Leu-Gly-Tyr-His-Val-Arg or -Gly-Ser-Tyr-Ala-Pro-Leu-Gly-Tyr-His-Val-Arg-Glu-Tyr-Pro-Ala-Gly-Val-Ser-Ala-Ala.

2. The new polypeptide according to claim 1, wherein the structure of the new polypeptide is shown as any one of TRF1-TRF12:

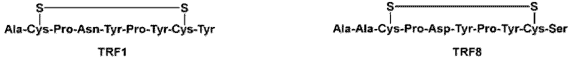

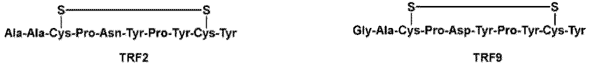

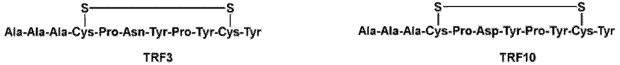

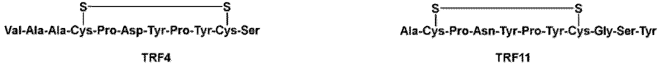

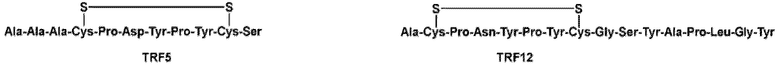

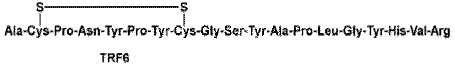

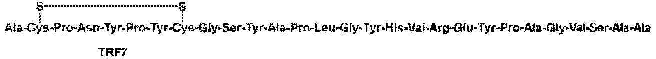

3. Use of the new polypeptide according to claim 1 in preparation of products for promoting tissue repair or improving aesthetic feeling of skin.

4. The use of the new polypeptide in preparation of products for promoting tissue repair or improving aesthetic feeling of skin according to claim 3, wherein the products are drugs, medical instruments or everyday chemicals.

5. The use of the new polypeptide in preparation of products for promoting tissue repair or improving aesthetic feeling of skin according to claim 4, wherein the drugs, the medical instruments or the everyday chemicals comprise an effective dose of one or more of the new polypeptides, or a pharmaceutically acceptable salt thereof or a stereoisomer thereof, with balance of auxiliary materials or other compatible drugs.

6. The use of the new polypeptide in preparation of products for promoting tissue repair or improving aesthetic feeling of skin according to claim 4, wherein the drugs, the medical instruments or the everyday chemicals are tablets, capsules, injections, liposome nanoparticles, controlled release agents, gel medicinal extracts, ointments, liniments for external use, patches, cream, detergents, lotion, gel, or toner.

7. The use of the new polypeptide in preparation of products for promoting tissue repair or improving aesthetic feeling of skin according to claim 5, wherein the auxiliary materials are solvents, disintegrants, flavoring agents, preservatives, colorants, or binders.

8. A drug composition, comprising the new polypeptide according to claim 1 or 2 and a pharmaceutically acceptable carrier.
